(19) Europäisches Patentamt / European Patent Office / Office européen des brevets

(11) **EP 3 961 513 A1**

(12) **EUROPEAN PATENT APPLICATION**

(43) Date of publication:
**02.03.2022 Bulletin 2022/09**

(51) International Patent Classification (IPC):
**G06N 3/04** *(2006.01)*   **G06N 3/08** *(2006.01)*

(21) Application number: **21184969.0**

(52) Cooperative Patent Classification (CPC):
**G06N 3/0454; G06N 3/084**

(22) Date of filing: **12.07.2021**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.08.2020 IN 202021036264**

(71) Applicant: **Tata Consultancy Services Limited
Maharashtra (IN)**

(72) Inventors:
• **SHARMA, ANKIT
  122003 Gurgaon, Haryana (IN)**

• **GUPTA, GARIMA
  122003 Gurgaon, Haryana (IN)**
• **PRASAD, RANJITHA
  122003 Gurgaon, Haryana (IN)**
• **CHATTERJEE, ARNAB
  122003 Gurgaon, Haryana (IN)**
• **VIG, LOVEKESH
  122003 Gurgaon, Haryana (IN)**
• **SHROFF, GAUTAM
  122003 Gurgaon, Haryana (IN)**

(74) Representative: **Goddar, Heinz J.
Boehmert & Boehmert
Anwaltspartnerschaft mbB
Pettenkoferstrasse 22
80336 München (DE)**

(54) **METHOD AND SYSTEM FOR CAUSAL INFERENCE IN PRESENCE OF HIGH-DIMENSIONAL COVARIATES AND HIGH-CARDINALITY TREATMENTS**

(57)    In presence of high-cardinality treatment variables, number of counterfactual outcomes to be estimated is much larger than number of factual observations, rendering the problem to be ill-posed. Furthermore, lack of information regarding the confounders among large number of covariates pose challenges in handling confounding bias. Essential is to find lower-dimensional manifold where an equivalent problem of causal inference can be posed, and counterfactual outcomes can be computed. Embodiments herein provide a method and system for CI in presence of high-dimensional covariates and high-cardinality treatments using Hi-CI DNN architecture comprising Hi-CI DNN model built by concatenating a decorrelation network and a modified regression network for jointly generating low-dimensional decorrelated covariates from the high-dimensional covariates, and predicting a set of outcomes for the input data set having the high-cardinality treatments comprising of the plurality of dosage levels by generating per-dosage level embedding to learn representation of the high-cardinality treatments.

FIG. 3

**Description**

CROSS-REFERENCE TO RELATED APPLICATIONS AND PRIORITY

The present application claims priority from Indian patent application No. 202021036264, filed on August 23, 2020.

TECHNICAL FIELD

The embodiments herein generally relate to machine learning based casual inference and, more particularly, to a Hi-CI (Hi-dimensional Causal Inference) Deep Neural Network (DNN) architecture for causal inference (CI) in presence of high-dimensional covariates and high-cardinality treatments.

BACKGROUND

Machine learning has enabled intelligent automation across different domains. Humans often justify several actions and events in terms of cause and effect. ML when applied for causal inferences has limitations since ML approaches are based on supervised learning techniques, where outcomes are strongly tied to the nature of training data. Thus, when such trained models are applied in real life scenarios, the real-time input data generating process may vary vastly, and hence these models do not generalize well to predict outcomes or inferences close to the real outcomes.
Efforts are made by researchers to integrate causality into machine learning models for obtaining robust and generalizable machine learning models. It is well-accepted that obtaining causal relations from an observational dataset is possible if underlying data generating process is well-understood. This is often posed as a problem of predicting the effects of interventions (or treatments) in the data generating process, and such treatments are generally enforced using policy or operational changes. Further, understanding the effect of intervention requires to accurately answer counterfactual or what-if type questions, which in turn necessitates modelling the causal relationship between the treatment and outcome variables.
Causal inference (CI) for observational studies lies at the heart of various domains like healthcare, digital marketing, econometrics-based applications, etc., that require quantifying the effect of a treatment or an intervention on an individual. As an example, consider a retail outlet optimizing the waiting time at a store since long queues leads to loss in customer base, in turn leading to low sales. In their historical observational data, consider the queue-length as a treatment variable and sale as an outcome variable. First, note that queue-length varies in the training data since it depends on the number of items purchased by every customer. A discount sale leads to a given customer buying more leading to higher queue-length. That is, training set includes examples with long queues and high sales. A naive supervised learning approach might incorrectly predict that increase in queue-length leads to increase in sales, whereas the true relationship between queue-length and sales is surely negative on regular days. Typically, with availability of information regarding discount sales, and including them in the model can correct for such effects. Such, variables affect both, the outcome, and the treatment, and hence, these variables are known as confounding covariates in the CI problem. Similarly, in a digital marketing context, age can be a confounding covariate which introduces selection bias in providing advertisements to young, middle-aged, and old-aged users and consequently a varying buying behavior (outcome). These aspects as well-captured in Simpson's paradox (Bottou et al., 2013), which states that the confounding behavior may lead to erroneous conclusions about causal relations and counterfactual estimation when the confounding variable is not considered in analysis. A key problem in modern empirical work is that datasets consists of large numbers of covariates (Newman, 2012) and high-cardinality treatments (Diemert et al., 2017). Thus, overall variations associated with real world data, which is to be processed to derive outcomes for CI scenarios may fall into different type of real-world scenarios such as 1) high-dimensional covariates, 2) high-cardinality treatments and 3) high-dimensional covariates with high-cardinality treatments with dosage levels. Specifically, in applications of healthcare, advertising etc., an individual's response plays an important role in guiding practitioners/observers to select the best possible interventions. Hence, it is essential to build ML models to handle such high-dimensional scenarios. Thus, when using ML for CI it is required to design machine learning models that abate confounding effects, while being parsimonious (simple models with great explanatory predictive power, which explain data with a minimum number of parameters, or predictor variables) in representation of high-dimensional variables, and adequately flexible.

SUMMARY

Embodiments of the present disclosure present technological improvements as solutions to one or more of the above-mentioned technical problems recognized by the inventors in conventional systems. For example, in one embodiment, a method for causal inference (CI) in presence of high-dimensional covariates and high-cardinality treatments is provided.

The method comprises building a High-dimensional Causal Inference Deep Neural Network (Hi-CI DNN) model for Causal Inference (CI) from an input data set comprising the high-dimensional covariates that are processed for the high-building a High-dimensional Causal Inference Deep Neural Network (Hi-CI DNN) model for Causal Inference (CI) from an input data set comprising the high-dimensional covariates that are processed for the high-cardinality treatments $(t_n(k))$ for a plurality of samples (n) of the input data set with cardinality *k*, wherein each of the high cardinality treatments comprising a plurality of dosage levels. The Hi-CI DNN model comprises: concatenating a decorrelation network and a modified regression network for jointly (i) generating low-dimensional decorrelated covariates from the high-dimensional covariates, and (ii) predicting a set of outcomes for the input data set having the high-cardinality treatments comprising of the plurality of dosage levels by generating per-dosage level embedding to learn representation of the high-cardinality treatments. The decorrelation network comprises an autoencoder employing a first loss function based on (i) a first component $\mathcal{L}_{ae}$ ($\Phi$, $\psi$) that minimizes a mean-squared loss between the low-dimensional decorrelated covariates, where $\Phi$ represents encoder of the autoencoder and $\Psi$ represents decoder of the autoencoder and the high-dimensional covariates, and (ii) a second component $\mathcal{L}_{ce}$ ($\Phi$), which is a cross entropy measure and a third component $\mathcal{L}_{2,1}$ ($M_D$) enabling confounding bias compensation to minimize disparity between factual treatments and counter factual treatments among the plurality of treatments, wherein $M_D$ is a matrix representing mixed norm on difference of means, and wherein the first loss function of the decorrelation network is represented by: where $\beta$, $\gamma$ are values obtained by hyperparameter tuning on validation datasets. The modified regression network comprising a plurality of embeddings $\Omega_e$ corresponding to the plurality of dosage levels and employing a second loss function comprising a root mean square error (RMSE) loss function and represented by:

$$\mathcal{L}_{\mathcal{RMSE}}(\text{y}, \hat{y}) =$$

$$\sqrt{\frac{1}{N}\sum_{n=1}^{N}\sum_{k=1}^{K}\sum_{e=1}^{E}\|y_n(k_e) - \hat{y}_n(k_e)\|^2} \, ,$$

wherein $y_n(k_e)$ is groundtruth and $\hat{y}_n(k_e)$ *is* set of outcomes predicted by the Hi-CNN model, and wherein $\hat{y}_n = \Omega_e([\Phi(x_n), t_n]^T)$.

Furthermore, the method comprises training the Hi-CI DNN model for predicting the set of outcomes for the input data set in accordance to an overall loss function of the Hi-CI DNN model, wherein the overall loss function jointly employs the first loss function and the second loss function and is represented by:

$$\mathcal{L}(\Phi, \Psi, \Omega_e, \beta, \gamma, \lambda) = \mathcal{L}_{\mathcal{D}}(\Phi, \Psi, \beta, \gamma) + \lambda\mathcal{L}_{\mathcal{RMSE}}(\text{y}, \hat{y}).$$

Furthermore, the method comprises predicting the set of outcomes for test data using the trained Hi-CNN DNN model.

In another aspect, a system for causal inference (CI) in presence of high-dimensional covariates and high-cardinality treatments is provided. The system comprises a memory storing instructions; one or more Input/Output (I/O) interfaces; and one or more hardware processors coupled to the memory via the one or more I/O interfaces, wherein the one or more hardware processors are configured by the instructions to build a High-dimensional Causal Inference Deep Neural Network (Hi-CI DNN) mode 1 for Causal Inference (CI) from an input data set comprising the high-dimensional covariates that are processed for the high-building a High-dimensional Causal Inference Deep Neural Network (Hi-CI DNN) model for Causal Inference (CI) from an input data set comprising the high-dimensional covariates that are processed for the high-cardinality treatments $(t_n(k))$ for a plurality of samples (n) of the input data set with cardinality *k*, wherein each of the high cardinality treatments comprising a plurality of dosage levels. The Hi-CI DNN model comprises: concatenating a decorrelation network and a modified regression network for jointly (i) generating low-dimensional decorrelated covariates from the high-dimensional covariates, and (ii) predicting a set of outcomes for the input data set having the high-cardinality treatments comprising of the plurality of dosage levels by generating per-dosage level embedding to learn representation of the high-cardinality treatments. The decorrelation network comprises an autoencoder employing a first loss function based on (i) a first component $\mathcal{L}_{ae}$ ($\Phi$, $\Psi$) that minimizes a mean-squared loss between the low-dimensional decorrelated covariates, where $\Phi$ represents encoder of the autoencoder and $\Psi$ represents decoder of the autoencoder and the high-dimensional covariates, and (ii) a second component $\mathcal{L}_{ce}$ ($\Phi$), which is a cross entropy measure and a third component $\mathcal{L}_{2,1}$ ($M_D$) enabling confounding bias compensation to minimize disparity between factual treatments and counter factual treatments among the plurality of treatments, wherein $M_D$ is a matrix representing mixed norm on difference of means, and wherein the first loss function of the decorrelation network is represented by:

$$\mathcal{L}_D(\Phi, \Psi, \beta, \gamma) = \mathcal{L}_{ce}(\Phi) + \beta\mathcal{L}_{ae}(\Phi, \Psi) + \gamma\mathcal{L}_{2,1}(M_D),$$ where $\beta$, $\gamma$ are values obtained by hyperparameter tuning on validation datasets. The modified regression network comprising a plurality of embeddings $\Omega_e$ corresponding to the plurality of dosage levels and employing a second loss function comprising a root mean square error (RMSE) loss function and represented by:

$$\mathcal{L}_{RMSE}(y, \hat{y}) = \sqrt{\frac{1}{N}\sum_{n=1}^{N}\sum_{k=1}^{K}\sum_{e=1}^{E}\|y_n(k_e) - \hat{y}_n(k_e)\|^2},$$

wherein $y_n(k_e)$ is groundtruth and $\hat{y}_n(k_e)$ *is* set of outcomes predicted by the Hi-CNN model, and wherein $\hat{y}_n = \Omega_e([\Phi(x_n), t_n]^T)$.

Furthermore, the system is configured to train the Hi-CI DNN model for predicting the set of outcomes for the input data set in accordance to an overall loss function of the Hi-CI DNN model, wherein the overall loss function jointly employs the first loss function and the second loss function and is represented by:

$$\mathcal{L}(\Phi, \Psi, \Omega_e, \beta, \gamma, \lambda) = \mathcal{L}_D(\Phi, \Psi, \beta, \gamma) + \lambda\mathcal{L}_{RMSE}(y, \hat{y}).$$

Furthermore, the system is configured to predict the set of outcomes for test data using the trained Hi-CNN DNN model

In yet another aspect, there are provided one or more non-transitory machine-readable information storage mediums comprising one or more instructions, which when executed by one or more hardware processors causes a method for causal inference (CI) in presence of high-dimensional covariates and high-cardinality treatments is provided. The method comprises building a High-dimensional Causal Inference Deep Neural Network (Hi-CI DNN) model for Causal Inference (CI) from an input data set comprising the high-dimensional covariates that are processed for the high-building a High-dimensional Causal Inference Deep Neural Network (Hi-CI DNN) model for Causal Inference (CI) from an input data set comprising the high-dimensional covariates that are processed for the high-cardinality treatments ($t_n(k)$) for a plurality of samples (n) of the input data set with cardinality *k*, wherein each of the high cardinality treatments comprising a plurality of dosage levels. The Hi-CI DNN model comprises: concatenating a decorrelation network and a modified regression network for jointly (i) generating low-dimensional decorrelated covariates from the high-dimensional covariates, and (ii) predicting a set of outcomes for the input data set having the high-cardinality treatments comprising of the plurality of dosage levels by generating per-dosage level embedding to learn representation of the high-cardinality treatments. The decorrelation network comprises an autoencoder employing a first loss function based on (i) a first component $\mathcal{L}_{ae}$ ($\Phi$, $\Psi$) that minimizes a mean-squared loss between the low-dimensional decorrelated covariates, where $\Phi$ represents encoder of the autoencoder and $\Psi$ represents decoder of the autoencoder and the high-dimensional covariates, and (ii) a second component $\mathcal{L}_{ce}$ ($\Phi$), which is a cross entropy measure and a third component $\mathcal{L}_{2,1}$ ($M_D$) enabling confounding bias compensation to minimize disparity between factual treatments and counter factual treatments among the plurality of treatments, wherein $M_D$ is a matrix representing mixed norm on difference of means, and wherein the first loss function of the decorrelation network is represented by:

$$\mathcal{L}_D(\Phi, \Psi, \beta, \gamma) = \mathcal{L}_{ce}(\Phi) + \beta\mathcal{L}_{ae}(\Phi, \Psi) + \gamma\mathcal{L}_{2,1}(M_D),$$ where $\beta$, $\gamma$ are values obtained by hyperparameter tuning on validation datasets. The modified regression network comprising a plurality of embeddings $\Omega_e$ corresponding to the plurality of dosage levels and employing a second loss function comprising a root mean square error (RMSE) loss function and represented by:

$$\mathcal{L}_{RMSE}(y, \hat{y}) =$$

$$\sqrt{\frac{1}{N} \sum_{n=1}^{N} \sum_{k=1}^{K} \sum_{e=1}^{E} \|y_n(k_e) - \hat{y}_n(k_e)\|^2} \, ,$$

wherein $y_n(k_e)$ is groundtruth and $\hat{y}_n(k_e)$ *is* set of outcomes predicted by the Hi-CNN model, and wherein $\hat{y}_n = \Omega_e([\Phi(x_n), t_n]^T)$.

Furthermore, the method comprises training the Hi-CI DNN model for predicting the set of outcomes for the input data set in accordance to an overall loss function of the Hi-CI DNN model, wherein the overall loss function jointly employs the first loss function and the second loss function and is represented by:

$$\mathcal{L}(\Phi, \Psi, \Omega_e, \beta, \gamma, \lambda) = \mathcal{L}_D(\Phi, \Psi, \beta, \gamma) + \lambda \mathcal{L}_{RMSE}(y, \hat{y}).$$

Furthermore, the method comprises predicting the set of outcomes for test data using the trained Hi-CNN DNN model.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1A is a functional block diagram of a system for causal inference (CI) in presence of high-dimensional covariates and high-cardinality treatments using a Hi-CI (Hi-dimensional Causal Inference) Deep Neural Network (DNN) architecture, in accordance with some embodiments of the present disclosure.
FIG. 1B is a high-level architecture of the Hi-CI DNN used by the system of FIG. 1A, in accordance to with some embodiments of the present disclosure.
FIG. 2 is flow diagram illustrating a method for causal inference (CI) in presence of high-dimensional covariates and high-cardinality treatments using the Hi-CI DNN architecture of the system of FIG. 1A, in accordance with some embodiments of the present disclosure.
FIG. 3 depicts the Hi-CI DNN architecture of the system of FIG. 1A, in accordance with some embodiments of the present disclosure.
FIGS. 4 and 5 depict evaluation results of the Hi-CI DNN architecture against state of art techniques, in accordance with some embodiments of the present disclosure.

It should be appreciated by those skilled in the art that any block diagrams herein represent conceptual views of illustrative systems and devices embodying the principles of the present subject matter. Similarly, it will be appreciated that any flow charts, flow diagrams, and the like represent various processes which may be substantially represented in computer readable medium and so executed by a computer or processor, whether or not such computer or processor is explicitly shown.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following claims.

Overall variations associated with real world data, which is to be processed to derive outcomes for CI scenarios may fall into different type of real-world scenarios such as 1) high-dimensional covariates 2) high-cardinality treatments and 3) high-dimensional covariates with high-cardinality treatments with dosage levels. Specifically, in applications of healthcare, advertising etc., an individual's response plays an important role in guiding practitioners/observers to select the best possible interventions. Hence, it is essential to build ML models to handle such high-dimensional scenarios. Thus, when using ML for CI it is required to design machine learning models that abate confounding effects, while being parsimonious in representation of high-dimensional variables, and adequately flexible. Few example real world scenarios that are needed to be considered while building ML models for better and better prediction of outcomes are mentioned below.

1. High-dimensional covariates: A typical characteristic of genomic data is the presence of vast number of covariates. For example, a problem of interest is to genetically modify the plant Arabidopsis thaliana to shorten the time to flowering (Buhlmann, 2013) since fast growing crops lead to better food production. In the corresponding dataset, there are 47 instances of the outcome time to flowering and 21; 326 genes which are construed as covariates. The goal is to causally infer the effects of a single gene intervention on the outcome, considering the other genes as the covariates. A similar (but less severe) situation is also seen in the popular The Cancer Genomic Atlas (TCGA) project (Weinstein etal., 2013) which is a repository that consists of gene expression values of 20547 genes of 9659 individuals. Here the goal is to measure the gene expression values for several treatment strategies like medication, 2 chemotherapy and surgery (Schwab et al., 2019), so that the best treatment regimen is chosen.

2. High-cardinality treatments: An example of the Criteo dataset is provided to motivate high cardinality treatments. Criteo dataset (Diemert et al., 2017) includes browsing related activities of users for interaction with 675 campaigns. In the causal setting, these campaigns are considered as treatments with campaign effect on buying as the outcome (Dalessandro et al., 2012).

3. High-dimensional covariates, high cardinality treatments with dosages: The popular NEWS datasets consists of news items represented by 2870 bag-of-word covariates. These news items are read by viewers on media devices. In causal setting, media devices act as treatments. Since the number of news items can vary from few tens to hundreds, varying but finite viewing time is considered as dosage levels, while the readers' opinion on different media devices is considered as outcome (Schwab et al., 2019). In the above applications of healthcare, advertising etc., an individual's response plays an important role in guiding practitioners to select the best possible interventions. Hence, it is essential to build models to handle such high-dimensional scenarios.

Treatment effect estimation in the presence of high-dimensional covariates is a well-explored topic in statistical literature on causal inference. In (Robins et al.,1994), the authors proposed techniques based on inverse probability of treatment weighting (IPTW), which is sensitive to the propensity score model (Fan et al., 2016). Propensity score estimation was improved by employing covariate balancing propensity scores (CBPS) in high-dimensions (Imai and Ratkovic, 2014; Guo et al., 2016; Fan et al., 2016). LASSO regression for high-dimensional CI was proposed in (Belloni et al., 2014). Approximate residual balancing techniques for treatment effect estimation in high-dimensions is proposed in (Athey et al., 2018). A common trait among these works is that they focus on estimating the average treatment effect (ATE) in the presence of a large number of covariates but are limited to settings with only two treatments. In (Schwab et al., 2019), high-cardinality treatments and continuous treatments have been considered. Typically, in the context of continuous treatments, a given treatment has been represented using multiple dosage levels (Schwab et al., 2019) to account for the exploding cardinality of the treatment set (as each dosage is a unique treatment in itself). In statistical literature, continuous dosages have been handled using propensity scores (Hirano and Imbens, 2004), doubly robust estimation methods (Kennedy et al., 2017), generalized CBPS score (Fong et al., 2018), using estimation frameworks for both treatment assignment and outcome prediction (Galagate, 2016). Modern deep neural networks (DNN) based methods employ matching or balancing techniques for compensating confounding bias. Existing DNN based architectures for the multiple treatment scenario as proposed in (Sharma et al., 2020; Schwab et al., 2018) have a severe limitation with respect to their architectures. They employ a separate regression network per treatment, and hence, these neural networks cannot be used in the presence of a large number of treatments. Furthermore, in the presence of high-dimensional covariates, it is essential to design a parsimonious, yet lossless representation of these covariates. In several works such as (Johansson et al., 2016; Shalit et al., 2017), a latent representation for covariates is learnt by minimizing the discrepancy distances of the control and treatment populations to compensate for confounding bias, in the presence of binary treatments. Since such a data representation is not lossless, this approach is not suitable in the presence of high-cardinality variables. An autoencoder is used to learn an unbiased lossless representation of covariates, uncorrelated with respect to the multiple, yet small number of treatment variables (Atan et al., 2018; Zhang et al., 2019). On the other hand, matching based DNN techniques and similar individuals with dissimilar treatments using propensity scores (Schwab et al., 2018; Sharma et al., 2020; Ho et al., 2007). Matching is often accomplished using nearest neighbor match (Ho et al., 2007), propensity score (Schwab et al., 2018) or generalized propensity score (Sharma et al., 2020). These techniques are computationally infeasible in the presence of high-cardinality treatment variables as good recipes for matching require spanning the entire dataset in search of alternate treatment variables while ensuring a balance in the number of individuals per treatment.

From the above analysis of work in the literature, it is identified that in presence of high-cardinality treatment variables, the number of counterfactual outcomes to be estimated is much larger than the number of factual observations, rendering the problem to be ill-posed. Furthermore, lack of information regarding the confounders among large number of covariates pose challenges in handling confounding bias. Hence, it becomes essential to find a lower-dimensional manifold where an equivalent problem of causal inference can be posed, and counterfactual outcomes can be computed.

Embodiments herein provide a method and system for causal inference (CI) in presence of high-dimensional covariates and high-cardinality treatments using a High-dimensional Causal Inference (Hi-CI) Deep Neural Network (DNN) architecture. The Hi-CI DNN architecture comprises a Hi-CI DNN model built by concatenating a decorrelation network and a modified regression network for jointly i) generating low-dimensional decorrelated covariates from the high-dimensional covariates, and ii) predicting a set of outcomes for the input data set having the high-cardinality treatments comprising of the plurality of dosage levels by generating per-dosage level embedding to learn representation of the high-cardinality treatments. The Hi-CI DNN model abates confounding effects, while being parsimonious in representation of high-dimensional variables and is adequately flexible.

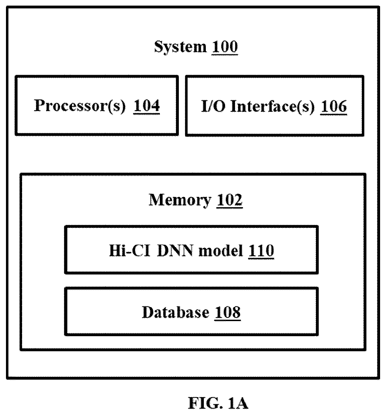

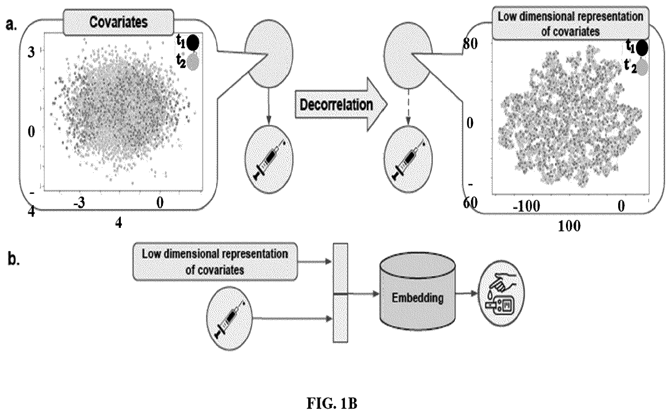

Referring now to the drawings, and more particularly to FIGS. 1A through 5, where similar reference characters denote corresponding features consistently throughout the figures, there are shown preferred embodiments and these embodiments are described in the context of the following exemplary system and/or method.

FIG. 1A is a functional block diagram of a system for causal inference (CI) in presence of high-dimensional covariates and high-cardinality treatments using a Hi-CI (Hi-dimensional Causal Inference) DNN architecture, in accordance with some embodiments of the present disclosure.

In an embodiment, the system 100, includes a processor(s) 104, communication interface device(s), alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100.

Referring to the components of the system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers and the like.

The I/O interface(s) 106 can include a variety of software and hardware interfaces, for example, a web interface, a graphical user interface, a touch user interface (TUI), voice interface and the like and can facilitate multiple communications within a wide variety of networks N/W and protocol types, including wired networks, for example, LAN, cable, etc., and wireless networks, such as WLAN, cellular, or satellite. In an embodiment, the I/O interface (s) 106 can include one or more ports for connecting a number of devices (nodes) of the system 100 to one another or to another server or devices.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes.

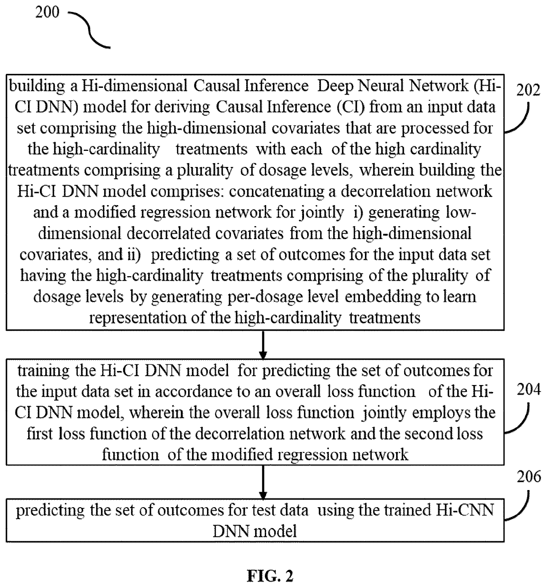

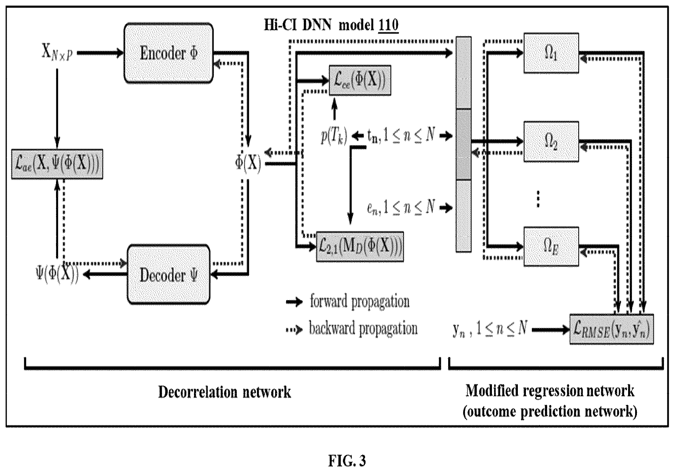

Further, the memory comprises a Hi-CI DNN model 110 built and trained by the system 100. The building of the Hi-CNN model 110 and the corresponding architecture is explained in conjunction with method of FIG. 2 and architecture in FIG. 3. The memory 102 may include a database 108, which may store the data generated, predicted outcomes of the system 100 and the like. The memory 102 may comprise information pertaining to input(s)/output(s) of each step performed by the processor(s) 104 of the system 100 and methods of the present disclosure. In an embodiment, the database 108 may be external (not shown) to the system 100 and coupled to the system via the I/O interface 106.

FIG. 1B is a high-level architecture of the Hi-CI DNN used by the system of FIG. 1A, in accordance with some embodiments of the present disclosure. FIG 1B (a) depicts a t-SNE plot on the left, while the right side depicts the decorrelated transformation of high-dimensional covariates in data, into a low-dimensional representation, using the Hi-CI DNN architecture, alternatively referred as Hi-CI framework or Hi-CI herein after. FIG. 1B (b) illustrates the dosage embedding to learn a lowdimensional representation of treatments followed by outcome prediction in the Hi-CI framework.

Thus, the Hi-CI framework disclosed herein enables obtaining an autoencoder based data representation for high-dimensional covariates while simultaneously handling confounding bias using a decorrelation loss. The Hi-CI framework caters to both, a large number of discrete, and continuous treatments, where a continuous treatment is characterized by a fixed number of dosage levels. The Hi-CI framework obtains a per-dosage level embedding layer to learn the low-dimensional representation of the high-cardinality treatments by jointly training the Hi-CI DNN model using root mean square (RMSE) loss and a sparsifying mixed norm loss function as depicted in part (b) of FIG. 1B.

FIG. 2 is flow diagram illustrating a method for causal inference (CI) in presence of high-dimensional covariates and high-cardinality treatments using the Hi-CI (Hi-dimensional Causal Inference) DNN architecture of system of FIG. 1A, in accordance with some embodiments of the present disclosure. In an embodiment, the system 100 comprises one or more data storage devices or the memory 102 operatively coupled to the processor(s) 104 and is configured to store instructions for execution of steps of the method 200 by the processor(s) or one or more hardware processors 104. The steps of the method 200 of the present disclosure will now be explained with reference to the components or blocks of the system 100 as depicted in FIG. 1A, architecture of the Hi-CNN model 110 as depicted in FIG. 3 and the steps of flow diagram as depicted in FIG. 2. Although process steps, method steps, techniques or the like may be described in a sequential order, such processes, methods and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

Referring to the steps of the method 200, at step 202, the one or more hardware processors 104 build the Hi-dimensional Causal Inference Deep Neural Network (Hi-CI DNN) model 110 which is executed by the one or more hardware processors 104, for Causal Inference (CI) from an input data set comprising the high-dimensional covariates that are processed for a high-cardinality treatments ($t_n(k)$), for a plurality of samples (n) of the input data set, with cardinality *k*, wherein each of the high cardinality treatments comprising a plurality of dosage levels (e).

Causal Inference Preliminaries required prior to building of the Hi-CI model 110 are mentioned below.

**The input dataset:** Also referred as the training data, $D_{CI}$ comprises of N samples from an observational dataset, where each sample is given by $\{x_n, y_n, t_n\}$, where $x_n \in X$. Each individual (also called context) n is represented using P covariates, i.e., $x_{np}$ denotes the $p^{th}$ covariate of the $n^{th}$ individual, for $1 \le n \le N$ Furthermore, an individual is subject to one of the K treatments given by $t_n = t_n(1), t_n(2) \dots t_n(K)$, where each entry of $t_n$ is binary, i.e., $t_n(k) \in \{0,1\}$. Here, $t_n(k) = 1$ implies that the kth treatment is provided. Assumed is that only one treatment is provided to an individual at any given point in time, and hence $t_n$ is a one-hot vector. A counterfactual is defined based on K-1 alternate treatments, and corresponding outcomes are referred to as counterfactual outcomes. Accordingly, the response vector for the n-th individual is given by $y_n \in \mathbb{R}^{K \times 1}$, i.e., the outcome is a continuous random vector with K entries denoted by $y_n(k)$, the response of the nth individual to the $k^{th}$ treatment. The set of counterfactual responses for the nth individual comprises of response to treatments $l \ne k$, given by $y_{n,1}$ and the size of this set is K-1. In the case of continuous treatment, assumed is that $t_n \in \mathbb{R}$ which implies that the treatment is a real-valued vector. However, to make the treatment set tractable the continuous treatment variable is casted using a finite set of E dosage levels (plurality of dosage levels) where E remains constant across treatments. Following the notation for discrete treatments, the outcome is a continuous random vector denoted by $y_n(k_e)$, where $1 \le k_e \le KE$, is the response of the $n^{th}$ individual to the $e^{th}$ dosage level of the $k^{th}$ treatment. In the case of discrete treatment, the maximum size of outcomes to be predicted by the Hi-CI DNN is N(K-1), while the number of available factual outcomes are N in number. It is evident that this problem is ill-posed when K is large. Furthermore, in the case of continuous treatments, effectively present are KE treatments, leading to N(KE-1) counterfactual responses. Considered here are observational studies where there are large number of covariates P and large number of treatments K. Goal is to train the Hi-CI DNN model 110 to overcome confounding and perform counterfactual regression, i.e., to predict the response, given any context and treatment, for large P and K. In the sequel, described are different components of the overall loss function that provides technical solution to manage confounding bias, high-dimensional treatments and high-dimensional covariates.

**Learning Representations from the input data set:** The crux of the loss function in CI for observational studies lie in techniques employed to compensate for the confounding bias. In this direction, the method disclosed employs autoencoders, which simultaneously encourage confounding bias compensation and learning compressed representation for the high-dimensional covariates. Alongside, employed is a Root Mean Square Error (RMSE) with mixed-norm regularizer based loss-function to obtain a low-dimensional representation for treatments. In the sequel, the mathematical constructs of learning the representation and the loss function are described.

Thus, referring back to step 202 of the method 200, building the Hi-CI DNN model 110 comprises: concatenating a decorrelation network and a modified regression network for jointly i) generating low-dimensional decorrelated covariates from the high-dimensional covariates, and ii) predicting a set of outcomes for the input data set having the high-cardinality treatments comprising of the plurality of dosage levels by generating per-dosage level embedding to learn representation of the high-cardinality treatments.

FIG. 3 depicts the Hi-CI DNN architecture of the system 100 of FIG. 1A, in accordance with some embodiments of the present disclosure. As depicted, the decorrelation network, executed by the one or more hardware processors 104, comprises an autoencoder employing a first loss function based on i) a first component $\mathcal{L}_{ae}$ ($\Phi$, $\Psi$) that minimizes a mean-squared loss between the low-dimensional decorrelated covariates and the high-dimensional covariates, where $\Phi$ represents encoder of the autoencoder and $\Psi$ represents decoder of the autoencoder, and ii) a second component $\mathcal{L}_{ce}$ ($\Phi$), which is a cross entropy measure and a third component $\mathcal{L}_{2,1}$ ($M_D$) enabling confounding bias compensation to minimize disparity between factual treatments and counter factual treatments among the plurality of treatments. All the loss functions and the related parameters are defined below. The autoencoder is used to jointly obtain a low-dimensional representation of the high-dimensional covariates and alleviate the effect of confounding. Let T represent the set of treatments, and $T_k \in$ T be a random variable; instantiation for the n-th individual is $t_n(k)$. Using an autoencoder, a mapping from the space of covariates $X$ such that $\Phi$: X $\to \mathcal{R}$ , where $\mathcal{R} \in \mathbb{R}$, is the representation space. The mapping $\Phi$ is such that,

1. The induced distribution of the treatments over $\mathcal{R}$ , which is denoted by p($T_k$|$\Phi$(X)) is free of confounding bias for all k.
2. The representation of $x_n$ under $\Phi$(.) for all n is lossless.
3. It maps higher-dimensional covariates in P to a low-dimensional space of size L, i.e., L < P.

A typical propensity score based matching approach addresses the issue of confounding bias by balancing the propensity score to obtain similar covariate distributions across treated populations. Mathematically, a sub-sample $X_s$ of the original sample is considered such that it ensures that the following condition holds:

$$p(T_1|X_s) = p(T_2|X_s) = \ldots\ldots = p(T_K|X_s) \quad (1)$$

Note that the condition stated above does not necessitate that treatment and covariates variables are uncorrelated. On the other hand, the loss function associated to the autoencoder imposes a far more stringent condition (Atan et al., 2018) such that

$$p(T_k|X) = p(T_k), \forall k \quad (2)$$

for the entire sample $D_{Cl}$. Autoencoders have been employed in the literature for addressing some of the tasks such as lossless data representation (Atan et al., 2018; Ramachandra, 2018). However, the method 200 disclosed herein provides an approach where an autoencoder is used to jointly accomplish the goals as specified above, and primarily, low-dimensional representations.

To ensure lossless data representation, the loss function associated with the autoencoder jointly minimizes the mean-squared error loss between the reconstructed and the original covariates, and the distance between the unbiased (p($T_k$)) and the biased treatment distributions (p($T_k$|$\Phi$(X))) for all $k$, while maintaining the resultant mapping in a lower-dimension as compared to the original covariates (L < P). These goals can be achieved by using the following loss function:

$$\mathcal{L}_1(\Phi, \Psi, \beta) = \mathcal{L}_{ce}\big(\Phi(X)\big) + \beta\mathcal{L}_{ae}\Big(\Phi(X), \Psi\big(\Phi(X)\big)\Big) \quad (3)$$

where, $\mathcal{L}_{ce}$ ($\Phi$) is the cross-entropy measure. The cross-entropy measure, alternatively referred as cross entropy loss, is directly proportional to the Kullback-Liebler divergence between the distributions in question, and hence it is an appropriate metric to minimize the divergence between p($T_k$) and p($T_k$|$\Phi$(X)) for all $k$. Accordingly, $\mathcal{L}_{ce}$ ($\Phi$) is given by:

$$\mathcal{L}_{ce}(\Phi) = -\sum_{T\in\mathbf{T}} p(T) \log\Big(p\big(T\big|\Phi(X)\big)\Big) \quad (4)$$

Furthermore, the loss term $\mathcal{L}_{ae}$ ($\Phi$, $\Psi$) is employed to minimize the mean-squared loss between the reconstructed and the original covariates in the autoencoder. Mathematically represented as,

$$\mathcal{L}_{ae}(\Phi, \Psi) = \frac{1}{PN} \sum_{n=1}^{N} \sum_{p=1}^{P} \left\| \left( x_{n,p} - (\Phi \circ \Psi)(x_{n,p}) \right) \right\|^2 \quad (5)$$

Where, $\Psi$ is the decoder mapping such that $\Psi: \mathcal{R} \rightarrow \mathbf{X}$ and $\circ$ is a composition operator, and L < P, which ensures that a low-dimensional, yet meaningful representation of the high-dimensional covariates is obtained. As a regularizer, employed is the mixed norm on the difference of means, represented using the matrix $M_D$. The columns of $M_D$ are given by $\mu_{D,(T_i,T_j)} = \frac{1}{LK(K-1)} \left( \mu_{T_i}(\Phi(X)) - \mu_{T_j}(\Phi(X)) \right)$, where $\mu_{T_i}(\Phi(X)) \in \mathbb{R}^{L \times 1}$ is the mean of represen $\Phi(X)$tation for all individuals in **X**, given by $\Phi(X)$ that undergo treatment $T_i$. Since all possible pairs of treatments $(T_i, T_j)$, for all $T_i$ and $T_j$ are considered, $M_D$ is of dimension $\mathbb{R}^{L \times (K(K-1))}$. The mixed norm regularizer on $M_D$, denoted as $\mathcal{L}_{2,1}(M_D)$, is as follows:

$$\mathcal{L}_{2,1}(M_D) = \sum_{u=0}^{K(K-1)} \sqrt{\sum_{v=0}^{L-1} |M_D(u,v)|^2} \quad (6)$$

wherein $M_D$ is a matrix representing mixed norm on difference of means It is defined as the sum over maximum mean discrepancies in terms of covariates between all treatment pairs.

Thus, combining equations 4, 5 and 6 the combined loss function (first loss function) of the decorrelation network is represented by:

$$\mathcal{L}_D(\Phi, \Psi, \beta, \gamma) = \mathcal{L}_{ce}(\Phi) + \mathcal{L}_{ae}(\Phi, \Psi) + \gamma \mathcal{L}_{2,1}(M_D) \quad (7)$$

The above objective function cannot be computed directly since both $p(T_k|\Phi(X))$ and $p(T_k)$ are unknown for any k. The estimates of $p(T_k)$ for $1 < k \le K$ K is given by (Atan et al., 2018):

$$p(T_k = t) = \frac{\sum_{n=1}^{N} \llbracket (t_n(k)=t) \rrbracket}{N} \quad (8)$$

Where, $\llbracket$ (.) is the indicator function. Essentially, $p(T_k)$ provides a count-based probability of k-th treatmnt. Further, the functional form of $p(T_k|\Phi(x_n))$ is assumed to be similar to logistic regression as below:

$$p(T_k|\Phi(x_n)) = \frac{\exp\left( (\theta_{T_k})^T \Phi(x_n) \right)}{\sum_{k=1}^{K} \exp\left( (\theta_{T_k})^T \Phi(x_n) \right)} \quad (9)$$

where $\theta_{T_k} \in \mathbb{R}^{L \times 1}$ are the per-treatment parameters of the logistic regression framework.

This results in a modified version of equation 4 and is given by

$$\mathcal{L}_{ce}(\Phi) = -\sum_{k=1}^{K} p(T_k) \left(\theta_{T_k}\right)^T \Phi(x_n) - \log\left( \sum_{k=1}^{K} \left(\theta_{T_k}\right)^T \Phi(x_n) \right) \quad (10)$$

Further, as depicted in FIG. 3, the modified regression network (also referred as prediction outcome network), executed by the one or more hardware processors 104 is concatenated to the decorrelation network. Further, comprises a plurality of embeddings $\Omega_e$ corresponding to the plurality of dosage levels (E).

**Embeddings for high-dimensional treatment:** The Hi-CI DNN model 110 is designed for datasets with large number of unique treatments. While a single bit is sufficient to represent binary treatments (Johansson et al., 2016), a one hot representation is used within the DNN to represent a categorical treatment for a given user (Sharma et al., 2020). In the presence of high-cardinality treatment variables, i.e., treatments with several unique categories, the size of the one-hot vector becomes unmanageable. Furthermore, DNN architectures that cater to multiple treatments often use a sub-divided network as in (Schwab et al., 2018) and (Schwab et al., 2019), with one branch per treatment. Such a branching network based DNN architecture becomes computationally intractable as the number of treatments increase.

An aspect that matters the most about one-hot encoding is the fact that one-hot mapping does not capture any similarity in treatment categories. For instance, if treatments $t_1$ and $t_2$ are drugs for lung-related issues, and $t_3$ is a treatment for skin-acne which is seemingly an unrelated issue, $t_1$, $t_2$ and $t_3$ are equidistant in the one-hot encoding space.

The Hi-CI DNN model 110 disclosed herein learns a representation of treatments denoted as $\Omega$: $[\Phi(\mathbf{X}), \mathbf{T}] \rightarrow \mathbf{Y}$, where $\Omega$ represents the space of output response vectors of length K, and the embedding encapsulates closeness property of treatments. Such representations of the treatment space are extremely relevant in the current day observational studies, as explained in the introduction (refer above section prior to the description of FIG. 1A). The impact of the embedding is realized in the outcome prediction part of the network (modified regression network). The loss on the outputs of the outcome prediction layer is the root mean square error (RMSE) is given by:

$$\mathcal{L}_{\mathcal{RMSE}}(y, \hat{y}) = \sqrt{\frac{1}{N} \sum_{n=1}^{N} \sum_{k=1}^{K} \| y_n(k) - \hat{y}_n(k) \|^2} \qquad (11)$$

where $\hat{y}_n = \Omega([\Phi(x_n), t_n]^T)$.

Although the impact of embedding is evident only in the above loss function, note that the training of the Hi-CI DNN framework incorporates all of the loss functions combined in (7) and (11). Intuitively, through the mixed norm based regularizer in (6), the distance between multiple populations is minimizes, whose covariate information is summarized by $\Phi(X)$ and hence, unable to exploit the similarity properties in the treatment itself. However, when the network is trained using equation (11) along with (6), in addition to promoting parsimonious representations owing to similarity of treatments, it is also ensured that such representation leads to a response close, in the sense of RMSE, to the true label.

**Modified Loss Function when E > 1 (for the modified regression network):** In the case of continuous treatment, a treatment is represented as consisting of multiple dosages (Schwab et al., 2019). In particular, it is assumed by the present disclosure that each treatment is specified by a set of E dosage levels, i.e., E remains constant across treatments. In the design of Hi-CIDNN, it is assumed that the treatment is affected by the confounding bias, but the dosage administered is not. However, since it is required to infer the per-dosage level counterfactual, exploited is the dosage information available in the labels $y_n(k_e)$. Accordingly, incorporated are the dosage levels in a generalized RMSE loss function of equation (11) to generate modified loss function (second loss function) comprising a root mean square error (RMSE) loss function and represented by:

$$\mathcal{L}_{\mathcal{RMSE}}(y, \hat{y}) = \sqrt{\frac{1}{N} \sum_{n=1}^{N} \sum_{k=1}^{K} \sum_{e=1}^{E} \| y_n(k_e) - \hat{y}_n(k_e) \|^2} \qquad (12)$$

wherein $y_n(k_e)$ is groundtruth and $\hat{y}_n(k_e)$ is the set of outcomes predicted by the Hi-CI model, where $\hat{y}_n = \Omega_e([\Phi(x_n), t_n]^T)$

Thus, it can be understood that for E=1, equation (12) gets transformed to equation (11).

Referring back to method 200 and with reference to the HI-CI DNN model built at step 202, at step 204, the one or more hardware processors 104 are configured to train Hi-CI DNN model 110 for predicting the set of outcomes for the input data set (training data)in accordance with an overall loss function of the Hi-CI DNN model 110. The loss function for HI-CI DNN jointly employs the first loss function and a second loss function and is represented by:

$$\mathcal{L}(\Phi, \Psi, \Omega, \beta, \gamma, \lambda) = \mathcal{L}_{\mathcal{D}}(\Phi, \Psi, \beta, \gamma) + \lambda \mathcal{L}_{\mathcal{RMSE}}(y, \hat{y}) \qquad (13)$$

where $\beta$, $\gamma$, $\lambda$ are values obtained by hyperparameter tuning on validation datasets.

However, in the case of continuous treatments, the structure of the regression network alone is modified. Thus, the loss function represented by equation (13) is modified to obtain the per-dosage level embedding, which is denoted as $\Omega_e$ (.), where $1 < e \leq E$. The concatenation of learned representation $\Phi(y_n)$, treatment vector $t_n$ is used as an input to the embedding layer. The dosage information is used to obtain a subdivided network, i.e., the DNN is split based on dosages and not treatments since E << K. The overall loss function of the Hi-CI DNN model 110 for continuous treatments is given by:

$$\mathcal{L}(\Phi, \Psi, \Omega_e, \beta, \gamma, \lambda) = \mathcal{L}_{\mathcal{D}}(\Phi, \Psi, \beta, \gamma) + \lambda\mathcal{L}_{\mathcal{RMSE}}(y, \hat{y}) \qquad (14)$$

The generalized architecture of the Hi-CI DNN framework with continuous treatments is as depicted in FIG. 3. For discrete treatments, E = 1, and hence one embedding sub-network $\Omega$ (.), is used instead of multiple sub-networks $\Omega_e$ (.) for outcome prediction. Dotted arrows highlight joint learning of the decorrelating network and the modified regression network (outcome prediction network).

Furthermore, at step 206 of the method 200, the one or more hardware processors 104 predict the set of outcomes for test data using the trained Hi-CNN DNN model.

**Experimental Set-up to demonstrate the efficacy in counterfactual regression of the Hi-CI DNN model.**

The results of the experimentation are reported on a synthetically generated dataset (Sun et al., 2015), and the semi-synthetic NEWS dataset (Johansson et al., 2016) for evaluation. Since a counterfactual outcome is not available, it becomes impossible to test CI algorithms in the context of counterfactual prediction. As a solution, data generating processes (DGP) are employed for demonstrating the results. In this section, the present disclosure describes the datasets employed as well as the corresponding DGPs employed for each dataset. Furthermore, the present disclosure describes the metrics used for evaluating the Hi-CI framework where E = 1, namely precision in estimation of heterogeneous effect (PEHE) (Shalit et al., 2017) and Mean Absolute Percentage Error (MAPE) over Average Treatment Effect (ATE) (Sharma et al., 2019). In the case of continuous treatments, i.e., for E > 1, the Hi-CI framework is evaluated using Mean Integrated Squared Error (MISE) and MAPE over ATE with dosage metric.

**Datasets and DGP employed for each dataset:**

A) Synthetic (Syn): A synthetic process described in (Sun et al., 2015) was used to generate data for both multiple treatment as well as continuous valued treatment scenario. The DGP gives the flexibility to simulate the counterfactual responses along with the factual treatments and responses, thereby helping in better evaluation of the Hi-CI DNN model. The generation process in (Sun et al., 2015) allows for 5 confounding covariates while the remaining P-5 covariates are non-confounding. The number of covariates P, data size N and cardinality of treatment set K are fixed according to the requirement of experiment and is described in detail experimental results later.

B) NEWS: The publicly available bag-of-words context covariates for NEWS dataset has been considered. The DGP as given in (Schwab et al., 2018) is employed for synthesizing one of multiple treatments and corresponding response for each document (context) in NEWS dataset. This generation process is extended to treatments with dosage levels by (Schwab et al., 2019) and is used for experimental evaluation of continuous valued treatments. The number of covariates P is fixed to 2870 and value for N, K is as obtained based on experimental requirements.

Convention of naming has been used for each newly synthesized dataset as a conjunction of the original dataset name and the treatment set cardinality (K) for all experiments performed. For example, 'NEWS4' denotes NEWS dataset for K = 4 treatment case.

**Metrics used for evaluating the Hi-CI DNN model:**

A) Precision in Estimation of Heterogeneous Effect (PEHE): The definition of PEHE as specified in (Schwab et al., 2018) is used for multiple treatments as:

$$\hat{\epsilon}_P = \frac{1}{\binom{K}{2}} \sum_{m=1}^{K} \sum_{r=1}^{m-1} \hat{\epsilon}_{P_{m,r}} \qquad (15)$$

$$\hat{\epsilon}_{P_{m,r}} = \frac{1}{N} \sum_{n=1}^{N} ([y_n(m) - y_n(r)] - [\hat{y}_n(m) - \hat{y}_n(r)])^2 \qquad (16)$$

where, $y_n(m)$ and $y_n(r)$ are the response of the $n^{th}$ individual to treatments $T_m$ and Tr respectively.

B) Mean Absolute Percentage Error (MAPE) over Average Treatment Effect (ATE): $MAPE_{ATE}$ is used as a metric to estimate error in predicting average treatment effect for high-cardinality treatments, and is given by:

$$MAPE_{ATE} = \left| \frac{ATE_{actual} - ATE_{pred}}{ATE_{actual}} \right| \qquad (17)$$

where

$$ATE_{actual} = \frac{1}{N} \sum_{n=1}^{N} \left( y_n(k) - \frac{1}{K-1} \sum_{l=1, l \neq k}^{K} y_n(l) \right) \qquad (18)$$

and $ATE_{pred}$ is obtained by replacing $y_n(k)$ in the above equation by its predicted value $\hat{y}_n$ for all k.

C) Mean Integrated Squared Error (MISE): For high cardinality treatments with dosages, MISE is used as a metric (as in (Schwab et al., 2019)). This is the squared error of dosage-response computed across the dosage levels and averaged over all treatments and entire population.

D) MAPE over ATE with dosage: Disclosed is a new metric $MAPE_{ATE}^{Dos}$ for high cardinality treatments with dosages. This metric is useful for evaluating effect of a dosage level for factual treatment as opposed to counterfactual treatments. It is given by:

$$MAPE_{ATE}^{Dos} = \left| \frac{ATE_{actual}^{Dos} - ATE_{pred}^{Dos}}{ATE_{actual}^{Dos}} \right| \qquad (19)$$

where,

$$ATE_{actual}^{Dos} = \frac{1}{E} \sum_{e=1}^{E} \left( \frac{1}{N_E} \sum_{n=1}^{N_E} \left( y_n(k_e) - \frac{1}{K-1} \sum_{l=1, l \neq k}^{K} y_n(l_e) \right) \right) \qquad (20)$$

**Baselines:** Following are DNN based approaches to baseline Hi-CI DNN model for high cardinality treatments:

a) O-NN: O-NN does not account for confounding bias, so the decorrelation network of Hi-CI I s by passed and X is directly passed to the outcome network.

b) Multi Mapreduced-based Backpropagation Neural Network (MultiMBNN): Matching and balancing based architecture proposed in (Sharma et al.,2020)

c) PM: Propensity based matching (Schwab et al., 2018) employed for counterfactual regression.

d) Deep-Treat+: Deep-Treat (Atan et al., 2018) learns bias removing network and policy optimization network independently to learn optimal, personalized treatments from observation data. In order to use Deep-treat as a baseline, the Deep-Treat is modified to Deep-Treat+ and jointly train decorrelation network obtained from Deep-Treat, and outcome network of Hi-CI (Hi-CI DNN) to baseline the approach of the present disclosure.

e) Dose-Response Network (DRNet): is a DNN based technique (Schwab et al., 2019) to infer counterfactual responses when treatments have dosage values. This is used to baseline Hi-CI continuous valued treatment case.

**Experimental Results:** Extensive experimentation has been performed using the Hi-CI DNN framework on Syn and NEWS datasets. The experimental evaluation is primarily aimed at evaluating the performance of Hi-CI DNN under three broad settings: high-cardinality treatments; continuous valued treatments and high number of covariates.

**A) High-cardinality treatments (E = 1)**

• Effect of increasing the cardinality of treatment set: Here, Hi-CI in scenarios where the cardinality of treatments increases, while E = 1 . With increase in K, sample size N is also proportionally increased to keep the average number of samples per treatment (given by N/K) constant. Table 1 reports the mean and standard deviation of the performance metrics PEHE; $MAPE_{ATE}$ for Syn and NEWS datasets. For both the datasets, performance errors increase with increase in K. In the case of Syn dataset, error in estimating ATE is much lower than NEWS dataset for very large number of treatments. This is because the number of covariates (perhaps confounding too) in NEWS dataset are of the order of 2000 whereas in Syn, the number of covariates are fixed to 10 with 5 confounding variables.

**Table-1**

| Data Set | $\sqrt{\hat{\epsilon}_P}$ | | $MAPE_{ATE}$ | |
|---|---|---|---|---|
| Syn35 | 3.6764 | 0.4037 | 0.074 | 0.0188 |
| Syn48 | 7.4350 | 0.1705 | 0.1494 | 0.0048 |
| Syn103 | 7.0612 | 0.5124 | 0.1681 | 0.0054 |
| Syn216 | 7.7069 | 0.1531 | 0.1943 | 0.0101 |
| NEWS35 | 7.6256 | 0.0243 | 0.393 | 0.0095 |
| NEWS48 | 8.2675 | 0.0522 | 0.4821 | 0.0105 |
| NEWS100 | 8.9334 | 0.6425 | 0.566 | 0.0245 |
| NEWS200 | 9.4679 | 0.8524 | 0.8924 | 0.0859 |

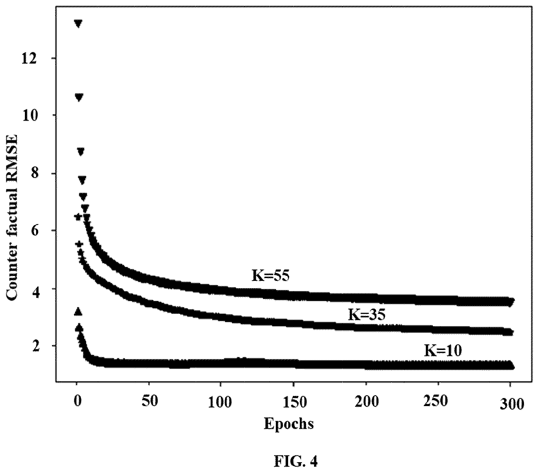

• Varying number of treatments K for fixed N: Illustrated is the performance of the Hi-CI framework keeping a sample size of N = 10000 while the cardinality of treatment set is varied from K = 10 to 100, which implies that there is a decrease in the ratio N/K. From Table 2, we observe that for Syn dataset, as the average number of samples per treatment decreases, PEHE and MAPEATE increase. However, for the NEWS dataset, no such trend is observed due to a large number but sparse covariates. Furthermore, FIG. 4 depicts the counterfactual RMSE for Syn datasets under this experimental setting. It is observed a slight increase in the counterfactual error as K increases, demonstrating that although the problem is harder, Hi-CI network prediction performs reasonably well.

**Table-2**

| Data Set | N/K | $\sqrt{\hat{\epsilon}_P}$ | $MAPE_{ATE}$ |
|---|---|---|---|
| Syn105 | 1000 | 1.6188,0.0262 | 0.046,0.008 |
| Syn35 | 285.7 | 3.6764,0.4037 | 0.074,0.0188 |
| Syn55 | 181.8 | 7.1836,0.8065 | 0.1378,0.0148 |
| Syn100 | 100 | 9.1706,0.8755 | 0.1812, 0.0129 |
| NEWS10 | 1000 | 7.8563,0.0214 | 0.6223,0.115 |
| NEWS35 | 285.7 | 7.6256,0.0243 | 0.393,0.0095 |
| NEWS55 | 181.8 | 7.7383,0.0273 | 0.4515,0.0360 |
| NEWS100 | 100 | 8.1432,0.0476 | 0.507,0.0171 |

• Loss Functions Analysis: Extensive experimentation was conducted to validate the impact of the disclosed decorrelation loss function $\mathcal{L}_{\mathcal{D}}$ (.) as given in equation (7), in learning the low-dimensional representation of data as the cardinality of treatments increases. The sample size was set to be constant while K increases, and consequently the ratio N/K decreases. From table 3A and table 3B (collectively referred as table 3), it is observed that PEHE and MAPE$_{ATE}$ decrease significantly when the lower-dimensional representation is learned using $\mathcal{L}_{\mathcal{D}}$ (.) loss function (7), a combination of losses that caters to reduction in bias via $\mathcal{L}_{ce}$ (.) reduction in information loss via $\mathcal{L}_{ae}$ (.), and similarity-exploiting via $\mathcal{L}_{2,1}$ (.) as compared where only $\mathcal{L}_1$ (.) or $\mathcal{L}_{a,e}(.) + \mathcal{L}_{2,1}(.)$ is used. Note that $\mathcal{L}_1$ (.) is considered as decorrelation loss in Deep-Treat+.

**Table-3A**

| Dataset | N/K | P | $\sqrt{\hat{\epsilon}_P}$ | | |
|---|---|---|---|---|---|
| | | | $\mathcal{L}_{1\,(.)}$ | $\mathcal{L}_{a,e}(.) + \mathcal{L}_{2,1}(.)$ | $\mathcal{L}_{\mathcal{D}\,(.)}$ |
| Syn105 | 1000 | 10 | 1.6390, 0.1125 | 1.6161, 0.0506 | 1.6188, 0.0262 |
| Syn35 | 285.7 | 10 | 5.3784, 0.4538 | 4.2283, 0.8902 | 3.6764, 0.4037 |
| Syn55 | 181.8 | 10 | 7.5039, 0.4699 | 7.5173, 0.5540 | 7.1836, 0.8065 |
| Syn100 | 100 | 10 | 9.7575, 0.7000 | 11.3353, 0.7624 | 9.1706, 0.8755 |
| NEWS10 | 1000 | 2870 | 7.8601, 0.0487 | 7.8541, 0.0285 | 7.8563, 0.0214 |
| NEWS35 | 285.7 | 2870 | 8.3121, 0.0442 | 8.3425, 0.0600 | 7.6256, 0.0243 |
| NEWS55 | 181.8 | 2870 | 7.8019, 0.0297 | 7.8212, 0.1648 | 7.7383, 0.0273 |
| NEWS100 | 100 | 2870 | 8.3275, 0.0792 | 8.2897, 0.0284 | 8.1432, 0.0476 |

**Table-3B**

| Dataset | N/K | P | $MAPE_{ATE}$ | | |
|---|---|---|---|---|---|
| | | | $\mathcal{L}_{1\,(.)}$ | $\mathcal{L}_{a,e}(.) + \mathcal{L}_{2,1}(.)$ | $\mathcal{L}_{\mathcal{D}\,(.)}$ |
| Syn105 | 1000 | 10 | 0.0645, 0.0243 | 0.0573, 0.0111 | 0.046, 0.008 |
| Syn35 | 285.7 | 10 | 0.1686, 0.0181 | 0.0990, 0.0407 | 0.074, 0.0188 |
| Syn55 | 181.8 | 10 | 0.1443, 0.0115 | 0.1472, 0.0103 | 0.1378, 0.0148 |
| Syn100 | 100 | 10 | 0.2214, 0.0394 | 0.2138, 0.0067 | 0.1812, 0.0129 |
| NEWS10 | 1000 | 2870 | 0.6288, 0.0146 | 0.6325, 0.0027 | 0.6223, 0.115 |
| NEWS35 | 285.7 | 2870 | 0.4875, 0.0141 | 0.4874, 0.0081 | 0.393, 0.0095 |
| NEWS55 | 181.8 | 2870 | 0.4792, 0.0173 | 0.6454, 0.0945 | 0.4515, 0.0360 |
| NEWS100 | 100 | 2870 | 0.5028, 0.0169 | 0.4844, 0.0050 | 0.507, 0.0171 |

**B) Varying number of covariates P:** The performance of the Hi-CI framework is illustrated by increasing the number of covariates, retaining the sample size fixed at N = 10000, i.e., P/N varies from 0:001 to 0:1. In the context of Syn35 dataset, it is observed from Table 4 that as the number of covariates increase, $\sqrt{\hat{\epsilon}_P}$ is as low as 3:67 and $MAPE_{ATE}$ is lower than 0:17, thereby showing the strength of the Hi-CI in handling high-dimensional covariates.

**Table-4**

| P/N | $\sqrt{\hat{\epsilon}_P}$ | $MAPE_{ATE}$ |
|---|---|---|
| 0.001 | 3.6764, 0.4037 | 0.074, 0.0188 |
| 0.005 | 5.1845, 0.7025 | 0.1388, 0.0192 |
| 0.01 | 6.2392, 0.3310 | 0.1557, 0.0132 |
| 0.05 | 6.0466, 0.4325 | 0.1720, 0.0104 |
| 0.1 | 6.2516, 0.6775 | 0.1757, 0.0260 |

**C) High-cardinality treatments with continuous dosages (E > 1):** In Table 5, the effect of varying number of dosage levels on the performance metrics for treatments with dosage is illustrated. Note that the error decreases as the number of dosage levels E increase. Measured is the dose-response error using MISE, and average dosage effect given by $MAPE_{ATE}^{Dos}$ in Table 5 shows that varying dosage levels does not impact the performance much. Note that this is partially, since context covariates are confounders for treatments, but not for dosage levels in the NEWS dataset. Furthermore, in case of synthetic dataset, although covariates are confounders for both treatments and dosages, it is observed that low-complexity networks are sufficient to capture the dosage-response. As mentioned, the Hi-CI DNN is designed under the assumption treatment is confounded but not dosage values. However, the results for Syn dataset, as seen in Table 5, show that Hi-CI disclosed can handle covariates confounding dosages as well.

**Table-5**

| Dataset | E | $\sqrt{\text{MISE}}$ | $MAPE_{ATE}^{Dos}$ |
|---------|----|--------------------|--------------------|
| Syn25 | 3 | 2.126, 0.0146 | 0.1193, 0.0024 |
| Syn25 | 6 | 1.980, 0.0157 | 0.1066, 0.0038 |
| Syn25 | 8 | 2.146, 0.014 | 0.124, 0.0021 |
| Syn25 | 10 | 3.148, 0.052 | 0.162, 0.0046 |
| NEWS25 | 3 | 11.2346, 0.1221 | 0.2462, 0.0584 |
| NEWS25 | 6 | 11.4860, 0.1568 | 0.3254, 0.1221 |
| NEWS25 | 8 | 11.0114, 0.0856 | 0.1457, 0.0462 |
| NEWS25 | 10 | 11.9086, 0.2795 | 0.6890, 0.1258 |

**Comparative analysis with baselines:** Illustrated is the performance of the Hi-CI network as compared to the popular baselines in literature.

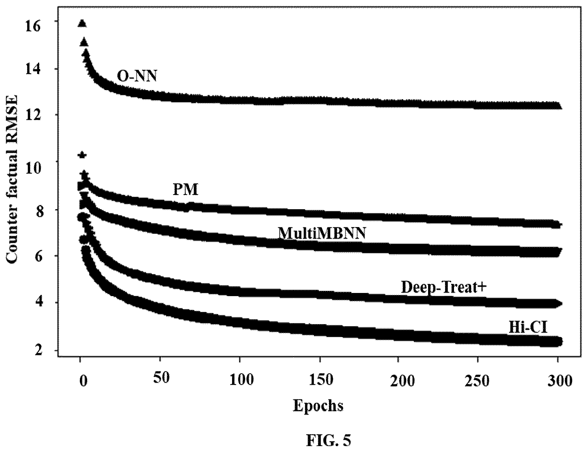

**A)** High-dimension treatments and covariates for E = 1: In table 6A and table 6B (collectively referred as table 6), depicted is the performance of Hi-CI framework as compared to the baselines with varying number of treatments for low and high-dimensional covariates. In order to evaluate the performance in high-dimensions, NEWS100 with P/N = 0:287 is shown to do exceedingly well in terms of both $\sqrt{\hat{\epsilon}_P}$ and $MAPE_{ATE}$, as compared to previous works. It is seen that for lower-cardinality treatment set (Syn4, NEWS4) Hi-CI based approach disclosed herein beats state of art marginally. This is expected behavior since baselines such as (Sharma et al., 2020) and (Schwab et al., 2018) are optimized for such scenarios. However, as the number of treatments increase, the Hi-CI outperforms baselines by huge margins. This behavior is observed for both high and low number of covariates. FIG. 5, depicts the counterfactual RMSE obtained using Hi-CI as compared to O-NN, PM, Deep-Treat+, indicating that Hi-CI framework outperforms the state of art approaches for CI.

**Table-6A**

| Dataset | P/N | N/K | $\sqrt{\hat{\epsilon}_P}$ | | |
|---------|-------|-------|-----------------|----------------|----------------|
| | | | PM | MultiMBNN | Hi-CI |
| Syn4 | 0.001 | 2500 | 1.9004, 0.1124 | 1.8272, 0.0928 | 1.3520, 0.0542 |
| Syn10 | 0.001 | 1000 | 0.4249, 0.1142 | 0.3917, 0.1075 | 0.0150, 0.0022 |
| Syn35 | 0.001 | 285.7 | 18.5894, 0.232 | 17.6520, 0.2032 | 3.6764, 0.4037 |
| Syn100 | 0.001 | 100 | 32.0424, 0.9862 | 32.304, 0.9652 | 9.1706, 0.8755 |
| NEWS4 | 0.287 | 2500 | 8.1842, 0.4202 | 7.6606, 0.4077 | 6.4120, 0.3016 |
| NEWS10 | 0.287 | 1000 | 9.1540, 0.0245 | 9.002, 0.0185 | 7.8563, 0.0214 |
| NEWS35 | 0.287 | 285.7 | 18.5894,0.2329 | 17.6520,0.2032 | 3.6764, 0.4037 |

(continued)

| Dataset | P/N | N/K | $\sqrt{\hat{\epsilon}_P}$ | | |
|---------|-----|-----|------|-----------|-------|
| | | | PM | MultiMBNN | Hi-CI |
| NEWS100 | 0.287 | 100 | 48.3878, 0.5620 | 49.6386, 0.8520 | 8.1432, 0.0476 |

**Table6B**

| Dataset | P/N | N/K | $MAPE_{ATE}$ | | |
|---------|-----|-----|------|-----------|-------|
| | | | PM | MultiMBNN | Hi-CI |
| Syn4 | 0.001 | 2500 | 0.4249,0.1142 | 0.3917,0.1075 | 0.0150, 0.0022 |
| Syn10 | 0.001 | 1000 | 5.8976,0.1175 | 5.7752,0.1100 | 1.6188, 0.0262 |
| Syn35 | 0.001 | 285.7 | 0.4726, 0.0562 | 0.4528, 0.0864 | 0.074, 0.0188 |
| Syn100 | 0.001 | 100 | 1.1225, 0.1585 | 1.2854, 0.2012 | 0.1812, 0.0129 |
| NEWS4 | 0.287 | 2500 | 0.3232, 0.0574 | 0.1622, 0.0381 | 0.0984, 0.0245 |
| NEWS10 | 0.287 | 1000 | 0.8641, 0.0962 | 0.7452, 0.105 | 0.6223, 0.115 |
| NEWS35 | 0.287 | 285.7 | 0.4726, 0.0562 | 0.4528, 0.0864 | 0.074, 0.0188 |
| NEWS100 | 0.287 | 100 | 1.9850, 0.1824 | 2.2014, 0.2350 | 0.507, 0.0171 |

**B)** High cardinality treatments with continuous dosages: In Table 7 depicted is the comparative dosage-response values for different datasets averaged over all treatments and individuals, in terms of $\sqrt{MISE}$. It is observed that the Hi-CI framework outperforms the state of the art DNN-based approach, DRNet by a considerable margin for several treatment counts. Table 7 compares with baselines the Hi-CI for continuous treatments, E > 1.

**Table-7**

| Dataset | DRNet | Hi-CI |
|---------|-------|-------|
| NEWS2 | 7.7, 0.2 | 6.2450, 0.1254 |
| NEWS4 | 11.5, 0.0 | 11.0842, 0.1358 |
| NEWS8 | 10.0, 0.0 | 8.7540, 0.1032 |
| NEWS16 | 10.2, 0.0 | 8.6560, 0.0452 |

An example implementation of the Hi-CI DNN model 110 is provided below. Algorithm 1 provides the methodology used for splitting input data set D into train ($D_{CI}$), validation ($D_{val}$), test ($D_{tst}$) sets. Also explained is the mechanism for hyperparameter selection. On the other hand, Algorithm 2 outlines the procedure for training Hi-CI DNN model 110 for the given set of hyperparameters. Parameters W of Hi-CI are initialized using random normal distribution. Adam optimizer with inverse time decay learning rate is used for gradient descent. In algorithm 1, *hparam values* specifies the range of hyperparameters for grid-search as in Table 8, *num_unique_treat(.)* returns the number of unique treatments in the dataset passed as argument, *get_gs_hparams(.)* returns set containing exhaustive combination of hyperparameters, *get_best_params(.)* returns Hi-CI parameters corresponding to best validation loss and *get_metric(.)* returns performance metrics of trained Hi-CI on dataset passed as argument. Similarly in algorithm 2, *initialize(.)* initializes parameters of Hi-CI using random normal distribution, *get_random_batches(.)* creates random batches of the dataset with batch size as specified in the argument, *train(.)* trains Hi-CI, *check_convergence(.)* checks for convergence on $D_{val}$, *get_final_params(.)* returns learned parameters $W_f$ of Hi-CI and *get_val_loss(.)* returns loss $\mathcal{L}$ on $D_{val}$ corresponding to $W_f$.

**Algorithm 1 Hi-CI:**

1: procedure Hi-CI (D, K, *hparam_values*, E = 1)

2:     Split D into $D_{CI}$ ,$D_{val}$,$D_{tst}$

3:     while *num_unique_treat*($D_{CI}$ ) < K do

4:         Split D into $D_{CI}$ ,$D_{val}$,$D_{tst}$

5:     $\mathcal{L}_{val} = \emptyset$ , W = $\emptyset$ ;

6:     *gs_hparams = get_gs_hparams(hparam values)*

7:     for *gs_hparam* in *gs_hparams* **do**

8:         $\mathcal{L}_{val}$, W ← *trainer(gs_hparam*, $D_{CI}$, $D_{val}$)

9:     W' = *get best params*($\mathcal{L}_{val}$, W)

10:    PEHE, $MAPE_{ATE}$ = *get_metric*($D_{tst}$, W')

11.    return PEHE, $MAPE_{ATE}$, W'

Parameter Tuning and Model Selection: The optimal parameters W' are selected for Hi-CI by performing an exhaustive grid-search on the hyperparameters values mentioned in Table 8.

**Algorithm 2** Train Hi-CI

1: procedure *trainer(gs_hparam*,$D_{CI}$,$D_{val}$)

2: W = *initialize()*

3: *total_epochs = gs_hparam.total epochs*

4: *batch_size = gs_pharam.batch size*

5: while epoch $<=$ *total_epochs* do

6:     $D_{batches} = get\_random\_batches$($D_{CI}$, *batch_size*)

7:     for $D_{batch}$ in $D_{batches}$ do

8:         W = *train*(W,$D_{batch}$, *gs_hparam*)

9:         if *check_convergence*(W,$D_{val}$) then

10:            $W_f$ = *get_final_params*(W)

11:            break

12:        *epoch = epoch + 1*

13:    $\mathcal{L} = get\_val\_loss$($W_f$ ,$D_{val}$)

14.    return $\mathcal{L}$

**Table 8**

| Parameter and corresponding Values |
|---|
| Batch size: 64, 128, 256, 512 |

(continued)

| Parameter and corresponding Values |
|---|
| Total epochs :1000 |
| Learning rate: 0.06, 0.08, 0.1, 0.12, 0.14, 0.16 |
| Learning rate decay: 0.6, 0.65, 0.7, 0.75 |
| No. of iterations per decay: 1, 2 |
| Train set split ratio: 0.6 |
| Validation set split ratio: 0.2 |
| Test set split ratio: 0.2 |
| No. of encoder layers: 1, 2, 3, 5, 7 |
| No. of decoder layers: 3, 4, 5, 6, 7, 8 |
| No. of outcome layers: 3, 4, 5, 6, 7, 8 |
| No. of hidden nodes in encoder layers: 100, 150, 200, 250 |
| No. of hidden nodes in decoder layers: 100, 175, 250, 325, 400 |
| No. of hidden nodes in outcome network: 100, 200, 250, 300, 400, 500 |
| L-2 regularization co-efficient for $\Phi$, $\Psi$, $\Omega_e$: 0.01, 0.001, 0.0001 |

**Learning $\theta_{T_k}$:** The multi-class logistic regression library of scikit-learn is used for learning ($\theta_{T_k}$) in equation (9). The range of hyperparameters for grid-search in logistic regression is given in Table 9.

**Table-9**

| Parameter: Values |
|---|
| Inverse of regularization strength: 0.001, 0.01, 0.1, 1, 10 |
| Solver: newton-cg, sag, saga, lbfgs |
| Tolerance for stopping criteria: 1e-4, 1e-2 |

In CI applications, one commonly encounters situations where there are large number of covariates and large number of treatments in real-world observational studies. The biggest hindrance in such a scenario is in inferring which of the covariates is the actual confounder among the large number of covariates. Furthermore, the complexity of the situation is enhanced since one needs to determine such confounding effects per treatment, for a large number of treatments. The method and system disclosed herein tackle these seemingly hard scenarios using a generalized Hi-CI framework. The approach disclosed is based on a fundamental assumption that the high-dimensional covariates are often sparse and can be represented in a low-dimensional space. An autoencoder is employed to represent covariates in a low-dimensional space, without losing much information in the original covariates. Alongside, also incorporated is a decorrelating loss function, which ensures that an equivalent representation of the covariate space with a reduced confounding bias is obtained. Furthermore, using the fact that often several treatments/interventions are perhaps similar, an embedding is used to obtain a low-dimensional representation of the treatment. In literature, continuous treatments are used, which system herein addresses by using per-dosage level embedding.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (200) for Causal Inference (CI) in presence of high-dimensional covariates and high-cardinality treatments, the method comprising:
   building (202), via one or more hardware processors, a High-dimensional Causal Inference Deep Neural Network (Hi-CI DNN) model executed by the one or more hardware processors, for Causal Inference (CI) from an input data set comprising the high-dimensional covariates that are processed for the high-cardinality treatments ($t_n(k)$), for a plurality of samples ($n$) of the input data set, with cardinality ($k$), wherein each of the high cardinality treatments comprising a plurality of dosage levels ($e$), and wherein building the Hi-CI DNN model comprises:
   concatenating a decorrelation network and a modified regression network for jointly (i) generating low-dimensional decorrelated covariates from the high-dimensional covariates, and (ii) predicting a set of outcomes for the input data set having the high-cardinality treatments comprising of the plurality of dosage levels by generating per-dosage level embedding to learn representation of the high-cardinality treatments, wherein

   a) the decorrelation network, executed by the one or more hardware processors, comprises an autoencoder employing a first loss function based on (i) a first component $\mathcal{L}_{ae}$ ($\Phi$, $\Psi$) that minimizes a mean-squared loss between the low-dimensional decorrelated covariates and the high-dimensional covariates, where $\Phi$ represents encoder of the autoencoder and $\Psi$ represents decoder of the autoencoder and (ii) a second component $\mathcal{L}_{ce}$ ($\Phi$), which is a cross entropy measure and a third component $\mathcal{L}_{2,1}$ ($M_D$) enabling confounding bias compensation to minimize disparity between factual treatments and counter factual treatments among the plurality of treatments, wherein $M_D$ is a matrix representing mixed norm on difference of means, and wherein the first loss function of the decorrelation network is represented by:

   $$\mathcal{L}_D(\Phi, \Psi, \beta, \gamma) = \mathcal{L}_{ce}(\Phi) + \beta\mathcal{L}_{ae}(\Phi, \Psi) + \gamma\mathcal{L}_{2,1}(M_D),$$ where $\beta$, $\gamma$ are values obtained by hyperparameter tuning on validation datasets; and

b) the modified regression network, executed by the one or more hardware processors, comprising a plurality of embeddings $\Omega_e$ corresponding to the plurality of dosage levels and employing a second loss function comprising a root mean square error (RMSE) loss function and represented by:

$$\mathcal{L}_{\mathcal{RMSE}}(y, \hat{y}) = \sqrt{\frac{1}{N} \sum_{n=1}^{N} \sum_{k=1}^{K} \sum_{e=1}^{E} \|y_n(k_e) - \hat{y}_n(k_e)\|^2} \, ,$$

wherein $y_n(k_e)$ is groundtruth and $\hat{y}_n(k_e)$ is the set of outcomes predicted by the Hi-CI model, and wherein

$$\hat{y}_n = \Omega_e([\Phi(x_n), t_n]^T);$$

and training (204), via the one or more hardware processors, the Hi-CI DNN model for predicting the set of outcomes for the input data set in accordance to an overall loss function of the Hi-CI DNN model, wherein the overall loss function jointly employs the first loss function and the second loss function and is represented by:

$$\mathcal{L}(\Phi, \Psi, \Omega_e, \beta, \gamma, \lambda) = \mathcal{L}_{\mathcal{D}}(\Phi, \Psi, \beta, \gamma) + \lambda \mathcal{L}_{\mathcal{RMSE}}(y, \hat{y}).$$

2. The method of claim 1, further comprising predicting (206) the set of outcomes for test data using the trained Hi-CNN DNN model.

3. The method of claim 1, further comprising evaluating the predicted set of outcomes enabling evaluation for high-cardinality treatments using a Mean Absolute Percentage Error (MAPE) over Average Treatment Effect (ATE) metric represented by:

$$MAPE_{ATE} = \left| \frac{ATE_{actual} - ATE_{pred}}{ATE_{actual}} \right|$$

where,

$$ATE_{actual}r = \frac{1}{N} \sum_{n=1}^{N} \left( y_n(k) - \frac{1}{K-1} \sum_{l=1, l \neq k}^{K} y_n(l) \right).$$

4. The method of claim 1, further comprising evaluating the predicted set of outcomes for a dosage level among the plurality of dosage levels for factual treatment as opposed to counterfactual treatments using a Mean Absolute Percentage Error (MAPE) over Average Treatment Effect (ATE) metric represented by:

$$MAPE_{ATE}^{Dos} = \left| \frac{ATE_{actual}^{Dos} - ATE_{pred}^{Dos}}{ATE_{actual}^{Dos}} \right|$$

where,

$$ATE_{actual}^{Dos} = \frac{1}{E} \sum_{e=1}^{E} \left( \frac{1}{N_E} \sum_{n=1}^{N_E} \left( y_n(k_e) - \frac{1}{K-1} \sum_{l=1, l \neq k}^{K} y_n(l_e) \right) \right).$$

5. A system (100) for Causal Inference (CI) in presence of high-dimensional covariates and high-cardinality treatments, the system (100) comprising:

a memory (102) storing instructions;
one or more Input/Output (I/O) interfaces (106); and

one or more hardware processors (104) coupled to the memory (102) via the one or more I/O interfaces (106), wherein the one or more hardware processors (104) are configured by the instructions to:

build a High-dimensional Causal Inference Deep Neural Network (Hi-CI DNN) model for Causal Inference (CI) from an input data set comprising the high-dimensional covariates that are processed for the high-cardinality treatments $(t_n(k))$, for a plurality of samples (n) of the input data set, with cardinality $k$, wherein each of the high cardinality treatments comprising a plurality of dosage levels, wherein the Hi-CI DNN model comprises: concatenating a decorrelation network and a modified regression network for jointly (i) generating low-dimensional decorrelated covariates from the high-dimensional covariates, and (ii) predicting a set of outcomes for the input data set having the high-cardinality treatments comprising of the plurality of dosage levels by generating per-dosage level embedding to learn representation of the high-cardinality treatments, wherein

a) the decorrelation network, executed by the one or more hardware processors, comprises an autoencoder employing a first loss function based on (i) a first component $\mathcal{L}_{ae}$ $(\Phi, \Psi)$ that minimizes a mean-squared loss between the low-dimensional decorrelated covariates, where $\Phi$ represents encoder of the autoencoder and $\Psi$ represents decoder of the autoencoder and the high-dimensional covariates, and (ii) a second

component $\mathcal{L}_{ce}$ $(\Phi)$, which is a cross entropy measure and a third component $\mathcal{L}_{2,1}$ $(M_D)$ enabling confounding bias compensation to minimize disparity between factual treatments and counter factual treatments among the plurality of treatments, wherein $M_D$ is a matrix representing mixed norm on difference of means, and wherein the first loss function of the decorrelation network is represented by:

$$\mathcal{L}_{\mathcal{D}}(\Phi, \Psi, \beta, \gamma) = \mathcal{L}_{ce}(\Phi) + \beta\mathcal{L}_{ae}(\Phi, \Psi) + \gamma\mathcal{L}_{2,1}(M_D),$$ where $\beta, \gamma$ are values obtained by hyperparameter tuning on validation datasets; and

b) the modified regression network, executed by the one or more hardware processors, comprising a plurality of embeddings $\Omega_e$ corresponding to the plurality of dosage levels and employing a second loss function comprising a root mean square error (RMSE) loss function and represented by:

$$\mathcal{L}_{\mathcal{RMSE}}(y, \hat{y}) = \sqrt{\frac{1}{N}\sum_{n=1}^{N}\sum_{k=1}^{K}\sum_{e=1}^{E}\|y_n(k_e) - \hat{y}_n(k_e)\|^2},$$

wherein $y_n(k_e)$ is groundtruth and $\hat{y}_n(k_e)$ is set of outcomes predicted by the Hi-CNN model, and wherein

$$\hat{y}_n = \Omega_e([\Phi(x_n), t_n]^T);$$

and

train the Hi-CI DNN model for predicting the set of outcomes for the input data set in accordance to an overall loss function of the Hi-CI DNN model, wherein the overall loss function jointly employs the first loss function and the second loss function and is represented by:

$$\mathcal{L}(\Phi, \Psi, \Omega_e, \beta, \gamma, \lambda) = \mathcal{L}_{\mathcal{D}}(\Phi, \Psi, \beta, \gamma) + \lambda\mathcal{L}_{\mathcal{RMSE}}(y, \hat{y}).$$

6. The system (100) of claim 5, wherein the one or more hardware processors (104) are further configured to predict the set of outcomes for test data using the trained Hi-CNN DNN model.

7. The system (100) of claim 5, wherein the one or more hardware processors are further configured to evaluate the predicted set of outcomes enabling evaluation for high-cardinality treatments using a Mean Absolute Percentage Error (MAPE) over Average Treatment Effect (ATE) metric represented by:

$$MAPE_{ATE} = \left|\frac{ATE_{actual} - ATE_{pred}}{ATE_{actual}}\right|$$

where,

$$\mathrm{ATE}_{\mathrm{actual}} r = \frac{1}{N}\sum_{n=1}^{N}\left(y_n(k) - \frac{1}{K-1}\sum_{l=1,l\neq k}^{K} y_n(l)\right).$$

8. The system of claim 5, wherein the one or more hardware processors are further configured to evaluate the predicted set of outcomes for a dosage level among the plurality of dosage levels for factual treatment as opposed to counterfactual treatments using a Mean Absolute Percentage Error (MAPE) over Average Treatment Effect (ATE) metric represented by:

$$\mathrm{MAPE}_{\mathrm{ATE}}^{\mathrm{Dos}} = \left|\frac{\mathrm{ATE}_{\mathrm{actual}}^{\mathrm{Dos}} - \mathrm{ATE}_{\mathrm{pred}}^{\mathrm{Dos}}}{\mathrm{ATE}_{\mathrm{actual}}^{\mathrm{Dos}}}\right|$$

where

$$\mathrm{ATE}_{\mathrm{actual}}^{\mathrm{Dos}} = \frac{1}{E}\sum_{e=1}^{E}\left(\frac{1}{N_E}\sum_{n=1}^{N_E}\left(y_n(k_e) - \frac{1}{K-1}\sum_{l=1,l\neq k}^{K} y_n(l_e)\right)\right).$$

9. One or more non-transitory machine-readable information storage mediums comprising one or more instructions, which when executed by one or more hardware processors causes a method for causal inference (CI) in presence of high-dimensional covariates and high-cardinality treatments, the method comprising:
building a High-dimensional Causal Inference Deep Neural Network (Hi-CI DNN) model executed by the one or more hardware processors, for Causal Inference (CI) from an input data set comprising the high-dimensional covariates that are processed for the high-cardinality treatments ($t_n(k)$), for a plurality of samples (n) of the input data set, with cardinality (*k*), wherein each of the high cardinality treatments comprising a plurality of dosage levels (e), and wherein building the Hi-CI DNN model comprises:
concatenating a decorrelation network and a modified regression network for jointly (i) generating low-dimensional decorrelated covariates from the high-dimensional covariates, and (ii) predicting a set of outcomes for the input data set having the high-cardinality treatments comprising of the plurality of dosage levels by generating per-dosage level embedding to learn representation of the high-cardinality treatments, wherein

    a) the decorrelation network, executed by the one or more hardware processors, comprises an autoencoder employing a first loss function based on (i) a first component $\mathcal{L}_{ae}$ ($\Phi$, $\Psi$) that minimizes a mean-squared loss between the low-dimensional decorrelated covariates and the high-dimensional covariates, where $\Phi$ represents encoder of the autoencoder and $\Psi$ represents decoder of the autoencoder and (ii) a second component $\mathcal{L}_{ce}$ ($\Phi$), which is a cross entropy measure and a third component $\mathcal{L}_{2,1}$ ($M_D$) enabling confounding bias compensation to minimize disparity between factual treatments and counter factual treatments among the plurality of treatments, wherein $M_D$ is a matrix representing mixed norm on difference of means, and wherein the first loss function of the decorrelation network is represented by:

$$\mathcal{L}_{\mathcal{D}}(\Phi, \Psi, \beta, \gamma) = \mathcal{L}_{ce}(\Phi) + \beta\mathcal{L}_{ae}(\Phi, \Psi) + \gamma\mathcal{L}_{2,1}(M_D),$$ where $\beta$, $\gamma$ are values obtained by hyperparameter tuning on validation datasets; and
    b) the modified regression network, executed by the one or more hardware processors, comprising a plurality of embeddings $\Omega_e$ corresponding to the plurality of dosage levels and employing a second loss function comprising a root mean square error (RMSE) loss function and represented by:

$$\mathcal{L}_{\mathcal{RMSE}}(y, \hat{y}) = \sqrt{\frac{1}{N}\sum_{n=1}^{N}\sum_{k=1}^{K}\sum_{e=1}^{E}\|y_n(k_e) - \hat{y}_n(k_e)\|^2},$$

wherein $y_n(k_e)$ is groundtruth and $\hat{y}_n(k_e)$ is the set of outcomes predicted by the Hi-CI model, and wherein

$$\hat{y}_n = \Omega_e([\Phi(x_n), t_n]^T);$$

and

training the Hi-CI DNN model for predicting the set of outcomes for the input data set in accordance to an overall loss function of the Hi-CI DNN model, wherein the overall loss function jointly employs the first loss function and the second loss function and is represented by:

$$\mathcal{L}(\Phi, \Psi, \Omega_e, \beta, \gamma, \lambda) = \mathcal{L}_{\mathcal{D}}(\Phi, \Psi, \beta, \gamma) + \lambda \mathcal{L}_{\mathcal{RMSE}}(y, \hat{y}).$$

System 100

Processor(s) 104    I/O Interface(s) 106

Memory 102

Hi-CI DNN model 110

Database 108

FIG. 1A

**FIG. 1B**

200

building a Hi-dimensional Causal Inference Deep Neural Network (Hi-CI DNN) model for deriving Causal Inference (CI) from an input data set comprising the high-dimensional covariates that are processed for the high-cardinality treatments with each of the high cardinality treatments comprising a plurality of dosage levels, wherein building the Hi-CI DNN model comprises: concatenating a decorrelation network and a modified regression network for jointly i) generating low-dimensional decorrelated covariates from the high-dimensional covariates, and ii) predicting a set of outcomes for the input data set having the high-cardinality treatments comprising of the plurality of dosage levels by generating per-dosage level embedding to learn representation of the high-cardinality treatments

202

training the Hi-CI DNN model for predicting the set of outcomes for the input data set in accordance to an overall loss function of the Hi-CI DNN model, wherein the overall loss function jointly employs the first loss function of the decorrelation network and the second loss function of the modified regression network

204

predicting the set of outcomes for test data using the trained Hi-CNN DNN model

206

FIG. 2

FIG. 3

FIG. 4

FIG. 5

Europäisches
Patentamt
European
Patent Office
Office européen
des brevets

## EUROPEAN SEARCH REPORT

Application Number

EP 21 18 4969

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANKIT SHARMA ET AL: "Hi-CI: Deep Causal Inference in High Dimensions", ARXIV.ORG, CORNELL UNIVERSITY LIBRARY, 201 OLIN LIBRARY CORNELL UNIVERSITY ITHACA, NY 14853, 22 August 2020 (2020-08-22), XP081746609, * abstract * * page 5 - page 12; figures 1, 2 * | 1-9 | INV. G06N3/04 G06N3/08 |
| A | ANKIT SHARMA ET AL: "MultiMBNN: Matched and Balanced Causal Inference with Neural Networks", ARXIV.ORG, CORNELL UNIVERSITY LIBRARY, 201 OLIN LIBRARY CORNELL UNIVERSITY ITHACA, NY 14853, 28 April 2020 (2020-04-28), XP081654221, * the whole document * | 1-9 | |
| A | ABDULLAHI UMAR I ET AL: "Counterfactual domain adversarial training of neural networks", 2017 INTERNATIONAL CONFERENCE ON THE FRONTIERS AND ADVANCES IN DATA SCIENCE (FADS), IEEE, 23 October 2017 (2017-10-23), pages 151-155, XP033296237, DOI: 10.1109/FADS.2017.8253217 [retrieved on 2018-01-09] * the whole document * | 1-9 | TECHNICAL FIELDS SEARCHED (IPC)<br><br>G06N |

The present search report has been drawn up for all claims

| Place of search | Date of completion of the search | Examiner |
|---|---|---|
| The Hague | 17 January 2022 | Tsakonas, Athanasios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202021036264

**Non-patent literature cited in the description**

- **WEINSTEIN et al.** *The Cancer Genomic Atlas (TCGA) project,* 2013